# FASCICULE DE BREVET EUROPEEN

(11) **EP 2 188 241 B1**
(45) Date de publication et mention de la délivrance du brevet: **20.07.2016**
(21) Numéro de dépôt: 08836385.8
(22) Date de dépôt: 11.09.2008
(51) Int. Cl.: C07C 45/52, C07C 51/25, C07C 47/22, C07C 57/04, B01J 27/185, C07C 253/26, C07C 255/08

(54) **PROCEDE DE FABRICATION D'ACROLEINE A PARTIR DE GLYCEROL**
VERFAHREN ZUR HERSTELLUNG VON ACROLEIN AUS GLYCEROL
PROCESS FOR MANUFACTURING ACROLEIN FROM GLYCEROL

(30) Priorité: 20.09.2007 FR 0757708
(43) Date de publication de la demande: 26.05.2010
(73) Titulaire: ARKEMA FRANCE, 92700 Colombes (FR)
(72) Inventeur: DUBOIS, Jean-Luc, F-69390 MILLERY (FR)
(74) Mandataire: Bonnel, Claudine
(86) Numéro de dépôt international: PCT/FR2008/051620
(87) Numéro de publication internationale: WO 2009/044081

(56) Documents cités:
- FR-A- 695 931
- FR-A- 2 245 604
- FR-A- 2 884 818
- US-A- 3 094 552
- US-A- 4 381 411
- HANYN AND T YANAGIBASHI T: "manufacture of acrolein", NIPPON KAGAKU KAISHI: JOURNAL OF THE CHEMICAL SOCIETY OF JAPAN, CHEMICAL SOCIETY OF JAPAN. TOKYO, JP, vol. 37, 1 janvier 1934 (1934-01-01), page 538, XP009100525, ISSN: 0369-4577

## Description

La présente invention concerne la production d'acroléine et/ou d'acide acrylique à partir de glycérol et a plus particulièrement pour objet un procédé de préparation d'acroléine par déshydratation du glycérol en présence d'un système catalytique à base d'oxyde de fer et de phosphore.

Les ressources d'origine fossile, telles que les coupes pétrolières, pour l'industrie chimique seront épuisées d'ici quelques dizaines d'années. Les ressources d'origine naturelle et renouvelable comme matières premières alternatives sont par conséquent de plus en plus étudiées.

L'acroléine, un important intermédiaire de synthèse pour l'industrie chimique est produit industriellement par oxydation en phase gazeuse du propylène par l'oxygène de l'air en présence de systèmes catalytiques à base d'oxydes mixtes. Le glycérol, issu d'huiles végétales dans la production de carburants biodiesel est une des voies envisagées comme substitut au propylène, le glycérol pouvant être soumis à une réaction de déshydratation catalytique pour produire l'acroléine. Un tel procédé permet de répondre ainsi au concept de chimie verte dans un cadre plus général de protection de l'environnement.

De nombreux systèmes catalytiques ont déjà fait l'objet d'études pour la réaction de déshydratation du glycérol en acroléine.

On connaît par le brevet FR 695.931 un procédé de préparation d'acroléine à partir de glycérine suivant lequel on utilise, comme catalyseurs, des sels acides ayant au moins trois fonctions acides ou des mélanges de ces sels. La préparation de ces catalyseurs consiste à imprégner par exemple par du phosphate de fer de la pierre ponce réduite en fragments de la grosseur d'un pois. Selon les enseignements du brevet, le rendement obtenu avec ce type de catalyseur est supérieur à 80 %.

L'article à la page 538 du Journal of the Society of Chemical Industry, Japan, 1934, 37, décrit la déshydratation d'une solution aqueuse de glycérol en acroléine au contact de phosphate de fer FePO₄ imprégné sur de la pierre ponce et maintenu à une température entre 400 et 460°C.

Dans le brevet US 2,558,520, la réaction de déshydratation est faite en phase gaz/liquide en présence de terres de diatomées imprégnées de sels d'acide phosphorique, en suspension dans un solvant aromatique. Un taux de conversion du glycérol en acroléine de 72,3% est obtenu dans ces conditions.

Le brevet US 5,387,720 décrit un procédé de production d'acroléine par déshydratation du glycérol, en phase liquide ou en phase gaz, à une température allant jusqu'à 340°C, sur des catalyseurs solides acides définis par leur acidité de Hammett. Les catalyseurs doivent avoir une acidité de Hammett inférieure à +2 et de préférence inférieure à -3. Ces catalyseurs correspondent par exemple à des matériaux siliceux naturels ou de synthèse, comme la mordénite, la montmorillonite, les zéolithes acides ; des supports, tels que des oxydes ou des matériaux siliceux, par exemple l'alumine (Al₂O₃), l'oxyde de titane (TiO₂), recouverts par des acides inorganiques, mono, di ou triacides ; des oxydes ou oxydes mixtes tels que l'alumine gamma, l'oxyde mixte ZnO-Al₂O₃, ou encore des hétéropolyacides. L'utilisation de ces catalyseurs permettrait de remédier au problème de formation de produits secondaires générés avec les catalyseurs de type phosphate de fer décrits dans le document FR 695.931 précité.

Selon la demande WO 06/087084, les catalyseurs solides fortement acides dont l'acidité de Hammett Ho est comprise entre -9 et -18 présentent une forte activité catalytique pour la réaction de déshydratation du glycérol en acroléine et se désactivent moins vite.

Cependant, les catalyseurs préconisés dans l'état de la technique pour produire de l'acroléine à partir de glycérol entraînent généralement la formation de sous-produits tels que l'hydroxypropanone, le propanaldéhyde, l'acétaldéhyde, l'acétone, des produits d'addition de l'acroléine sur le glycérol, des produits de polycondensation du glycérol, des éthers de glycérol cycliques, mais aussi du phénol et des composés polyaromatiques qui sont à l'origine de la formation de coke sur le catalyseur et donc de sa désactivation. La présence des sous-produits dans l'acroléine, notamment de propanaldéhyde, pose de nombreux problèmes de séparation de l'acroléine et nécessite des étapes de séparation et purification qui conduisent à des coûts élevés de récupération de l'acroléine purifiée. De plus, lorsque l'acroléine est utilisée pour la production d'acide acrylique, le propanaldéhyde présent peut s'oxyder en acide propionique qui est difficile à séparer de l'acide acrylique notamment par distillation. Ces impuretés présentes réduisent fortement le champ d'application de l'acroléine produite par déshydratation du glycérol.

La Société Déposante a donc cherché à améliorer la production d'acroléine à partir de glycérol, en utilisant des catalyseurs plus sélectifs permettant d'obtenir des rendements élevés en acroléine, et présentant une activité sur de longues durées.

On connaît par ailleurs de nombreuses applications à des catalyseurs composés essentiellement de phosphate de fer. Parmi ces applications, on peut citer notamment :
- FR1 604 884 : Oxydation des oléfines et dioléfines
- US 3,725,494 : Procédé de production de dioléfines
- JP10-287610 : Production de méthylglyoxal par oxydation d'hydroxyacétone.
- JP2003-146935 : Préparation d'acide pyruvique par oxydation en phase vapeur de l'acide lactique.
- US 3,314,760 : Production de chlorométhane par oxychloration de méthane.
- US 3,173,962 : Production d'oléfines et d'hydrocarbures chlorés.
- GB 616 260 : Polymérisation et condensation d'oléfines.
- US 3,142,697 : Production de nitriles par ammoxydation d'une oléfine, et plus spécifiquement production d'acrylonitrile et methacrylonitrile.
- JP8-295687 : Production d'anhydride citraconique à partir d'acide lactique
- FR 2 499 557 : Production d'esters d'alkyles par oxydéshydrogénation-estérification de mélanges d'acides carboxyliques et d'alcools.
- US 4,381,411 : Oxydéshydrogénation d'aldéhydes saturés en aldéhydes insaturés et plus spécifiquement pour la production d'acroléine et de méthacroléine.

L'utilisation de systèmes catalytiques à base de phosphate de fer pour l'oxydéshydrogénation d'acides carboxyliques saturés en acides carboxyliques insaturés, en particulier la conversion de l'acide isobutyrique en acide méthacrylique a été largement décrite :
On peut citer la demande de brevet FR 2,514,756 qui décrit la réaction d'oxydéshydrogénation de l'acide isobutyrique en présence d'un phosphate de fer calciné contenant un métal extrinsèque, tel que l'argent, le lanthane, le cobalt ou le tellure, comme composant modificateur ou dopant.

Dans la demande de brevet FR 2 497 795, la même réaction est effectuée en présence d'un phosphate de fer modifié par la présence d'un métal choisi parmi le bore, l'aluminium, le gallium et l'indium. Il a été trouvé que le catalyseur au phosphate de fer modifié par l'aluminium est actif pendant de longues durées pour la réaction d'oxydéshydrogénation de l'acide isobutyrique.

La demande de brevet FR 2 245 604 décrit un procédé de préparation d'acides alpha, bêta-insaturés par déshydrogénation oxydante due à l'action d'oxygène ou de gaz contenant de l'oxygène, d'acides carboxyliques aliphatiques saturés en présence d'un catalyseur contenant, en combinaison avec l'oxygène, du fer, du phosphore et éventuellement un ou plusieurs éléments appartenant à la liste formée par lithium, sodium, potassium, rubidium, césium, magnésium, calcium, strontium, baryum, étain et antimoine. Cette composition catalytique peut être employée à l'exclusion de tout support ou en combinaison avec un support.

On connaît aussi par les brevets US-A-4 364 856 et US-A-4 473 707, un catalyseur à base d'oxyde de fer et de phosphore, qui se présente sous une forme enrobée sur un support. Un tel catalyseur est désigné ci-après par l'expression « catalyseur enrobé ». Son procédé de préparation consiste à mouiller partiellement un support, tel que la silice, avec une solution ou suspension colloïdale de SiO₂ dans l'eau; mettre le support partiellement mouillé en contact avec une poudre du catalyseur; et agiter le mélange ainsi réalisé pour former le catalyseur enrobé, lequel est ensuite séché, puis calciné. Le catalyseur proprement dit est représenté par la formule empirique AₐFe_{b}P_{c}D_{d}Oₓ, où :
- A est choisi parmi Al, B, Be, Cd, Co, Cr, Ga, Ge, In, Ni, Te, Th, Ti, Tl, U, V, Zn, Zr, les terres rares et leurs mélanges;
- D est choisi parmi Ag, Cu, Mn et leurs mélanges ; et
- a = 0-1,0; b = 0,75-1,5; c = 1,0-2,0; d = 0-2,0 ; a + d est supérieur à 0; et x est le nombre d'oxygène nécessaire pour satisfaire les exigences de valence des éléments restants.

On connaît également, par le brevet CA-A-1 186 673, un système catalytique à deux composants, comprenant, en mélange physique, un catalyseur du type fer/phosphate, et un support inerte (silice) dopé au phosphate, ledit support étant composant métallique extrinsèque dans la préparation du catalyseur facilite la formation de l'espèce à activité catalytique. Ce catalyseur présente l'avantage d'être actif vis à vis du produit secondaire propanaldéhyde éventuellement formé lors de la déshydratation du glycérol pour le transformer en acroléine et ainsi conduire à des sélectivités et rendements excellents.

La présente invention a donc pour objet un procédé de fabrication d'acroléine à partir de glycérol caractérisé en ce que l'on effectue la réaction de déshydratation du glycérol en présence d'un système catalytique comprenant un catalyseur répondant à la formule générale FePₓM'_{y}M"_{w}O_{z} dans laquelle :
- M' représente au moins l'un des éléments suivants: métal alcalin, métal alcalino-terreux, Al, B, Co, Ni, Sn, Sb, Mn, Ag, seuls ou en mélange, M" représente au moins l'un des éléments suivants: Pt, Rh, seuls ou en mélange,
- x allant de 0,2 à 3,0, bornes comprises, de préférence allant de 0,9 à 2,0
- y allant de 0,01 à 2,0, bornes comprises
- w allant de 0,0 à 0,1, bornes comprises
- z est la quantité d'oxygène liée aux autres éléments et correspondant à leur état d'oxydation.

Les éléments Cs, Al, B, Ag sont préférés comme métal M'.

Le système catalytique dans le procédé selon l'invention peut être massique et est dans ce cas utilisé à l'exclusion de tout support conduisant tel quel à une activité catalytique excellente.

En tant que composés de départ pour l'obtention du catalyseur du procédé de la présente invention, il est possible d'utiliser par exemple pour les composés de fer : nitrates, chlorures, sulfates, carbonates, sels d'acides organiques mono ou polycarboxyliques, chélates, etc. En tant que composés à base de phosphore, on peut utiliser les phosphates alcalins, phosphates d'ammonium, et les acides phosphoriques et phosphoreux, etc. En tant que composés des métaux composant métallique extrinsèque dans la préparation du système catalytique facilite la formation de l'espèce à activité catalytique. Ce système catalytique présente l'avantage d'être actif vis à vis du produit secondaire propanaldéhyde éventuellement formé lors de la déshydratation du glycérol pour le transformer en acroléine et ainsi conduire à des sélectivités et rendements excellents.

La présente invention a donc pour objet un procédé de fabrication d'acroléine à partir de glycérol caractérisé en ce que l'on effectue la réaction de déshydratation du glycérol en présence d'un système catalytique comprenant de l'oxygène, du fer, du phosphore, et un ou plusieurs éléments choisis parmi les métaux alcalins, les métaux alcalino-terreux, Al, Si, B, Co, Cr, Ni, V, Zn, Zr, Sn, Sb, Ag, Cu, Nb, Mo, Y, Mn, Pt, Rh, les terres rares La, Ce, Sm.

De préférence le système catalytique comprend un catalyseur répondant à la formule générale FePₓM'_{y}M"_{w}O_{z} dans laquelle :
- M' représente au moins l'un des éléments suivants: métal alcalin, métal alcalino-terreux, Al, B, Co, Ni, Sn, Sb, Mn, Ag, seuls ou en mélange, M" représente au moins l'un des éléments suivants: Pt, Rh, seuls ou en mélange,
- x allant de 0,2 à 3,0, bornes comprises, de préférence allant de 0,9 à 2,0
- y allant de 0,01 à 2,0, bornes comprises
- w allant de 0,0 à 0,1, bornes comprises
- z est la quantité d'oxygène liée aux autres éléments et correspondant à leur état d'oxydation.

Les éléments Cs, Al, B, Ag sont préférés comme métal M'.

Le système catalytique dans le procédé selon l'invention peut être massique et est dans ce cas utilisé à l'exclusion de tout support conduisant tel quel à une activité catalytique excellente.

En tant que composés de départ pour l'obtention de la composition catalytique du procédé de la présente invention, il est possible d'utiliser par exemple pour les composés de fer : nitrates, chlorures, sulfates, carbonates, sels d'acides organiques mono ou polycarboxyliques, chélates, etc. En tant que composés à base de phosphore, on peut utiliser les phosphates alcalins, phosphates d'ammonium, et les acides phosphoriques et phosphoreux, etc. En tant que composés des métaux alcalins ou alcalino-terreux, il est possible d'utiliser par exemple les composés suivants : nitrates, oxydes, hydroxydes, chlorures, sulfates, carbonates, bicarbonates, nitrites, phosphates, silicates, ainsi que les sels d'oxyacides ou d'acides mono - ou polycarboxyliques organiques tels que formiates, oxalates, citrates, tartrates, etc. En tant que composés des autres éléments, il est possible d'utiliser par exemple les oxydes, halogénures, sulfates, sels d'acides mono- ou polycarboxyliques organiques, etc.

Le catalyseur peut être préparé selon n'importe quelle méthode déjà connue, toutes les méthodes de préparation comportant une étape d'activation finale de la composition catalytique, laquelle consiste généralement en une calcination à une température comprise entre 350 et 1000°C.

Les méthodes les plus courantes comprennent la préparation d'une composition intégrale avant la calcination. Ceci peut être effectué facilement en utilisant la méthode dite à la bouillie ou la méthode de précipitation. Dans cette dernière méthode, on prépare d'abord une solution aqueuse de sels des métaux considérés et d'acide phosphorique et on neutralise ensuite avec une base appropriée pour précipiter les phosphates métalliques mixtes. On a avantage à laver soigneusement le précipité pour séparer toutes les traces de substances solubles dans l'eau et ensuite à sécher ayant la calcination. Dans l'alternative, on peut ajouter du phosphate d'ammonium à la solution des sels métalliques pour précipiter directement les phosphates métalliques. On peut utiliser n'importe quels sels de fer ou de métal solubles dans l'eau. Cependant en raison des caractéristiques de solubilité des nitrates, ces sels sont préférés.

La méthode dite à la bouillie est aussi bien appropriée pour préparer les catalyseurs utilisés dans le procédé de l'invention. Selon ce mode opératoire, on obtient la solution aqueuse des sels de fer et de métal considéré conjointement avec l'acide phosphorique. On chauffe la solution en continu jusqu'à ce que la masse ne puisse plus être agitée. On fragmente ensuite le résidu et on le chauffe à nouveau à une température moyennement élevée de l'ordre d'environ 120 °C jusqu'à séchage complet. Ensuite, on tamise le produit et on le calcine.

Selon l'une ou l'autre de ces techniques, on peut préparer un catalyseur sur support. Il est possible d'utiliser en tant que support toute matière telle que silice, alumine, oxyde de titane, carbure de silicium, mélange silice-alumine, silicates, borates, carbonates à condition que ces produits soient stables aux conditions réactionnelles auxquelles le catalyseur sera soumis. Le support est ajouté avant d'éliminer la teneur en eau. Le support peut également être imprégné dans une solution du phosphate métallique et séché. Les étapes d'imprégnation et séchage doivent être répétées jusqu'à ce que l'on obtienne le nombre désiré de couches de phosphate métallique séché. De manière semblable, dans la méthode alternative décrite, on peut effectuer la précipitation des phosphates métalliques en présence de particules en suspension du support prévu. On peut utiliser ensuite une technique de séchage par pulvérisation dans laquelle la bouillie est introduite dans le séchoir par pulvérisation et le produit solide est ensuite mis en comprimés ou extrudé puis calciné.

D'autres méthodes telles que celles indiquées ci-après peuvent aussi être utilisées pour préparer les catalyseurs utilisables dans l'invention :
- Les sels de composés alcalins ou alcalino-terreux sont dissous dans de l'acide phosphorique concentré, puis on ajoute le nitrate de fer hydraté. La solution est ensuite évaporée, le solide récupéré est séché, puis broyé et tamisé. On ajoute alors 1 % en poids de noir de carbone avant de pastiller le solide sous la forme de pastilles cylindriques creuses par exemple de 3,5 mm de haut, de diamètre intérieur de 1,5 mm, de diamètre extérieur de 5 mm. Le solide est finalement calciné à 460 °C.
- On dissout, dans un solvant tel que l'eau, la quantité désirée d'un composé contenant du fer. On y incorpore la quantité appropriée de phosphore sous la forme d'un acide ou d'une solution de sel dissous. De la silice colloïdale peut également être ajoutée jusqu'à 15% en poids par rapport au mélange résultant afin de donner au catalyseur la résistance physique souhaitée. Le pH de la solution est ensuite ajusté à 7 par l'addition d'une base, ce qui conduit à la formation d'un précipité de catalyseur brut fer/phosphate qui est séché après lavage, après combinaison avec des sels de métaux alcalins ou alcalino-terreux, ou autre élément dopant envisagé. En variante, on peut ajouter les sels de métaux alcalins ou alcalino-terreux à la solution de sel de fer et de phosphore avant la neutralisation. Après séchage, le catalyseur est broyé à la finesse désirée, puis calciné.

Dans un mode de réalisation particulier de l'invention, le catalyseur peut être associé à un support macroporeux, imprégnable à coeur, la matière active étant déposée à la surface de tous les pores du support. Le support de catalyseur peut également être additionné d'un phosphate en mélange physique avec la matière active contenant le phosphate de fer.

Le rapport molaire entre le phosphore et la matière de support inerte peut varier dans de larges limites. En général, le rapport P/support peut varier de 20:1 à 1:30. Ce rapport naturellement varie selon le type de la matière de support, en tenant compte que celle-ci doit conférer la résistance mécanique au catalyseur et augmenter sa surface spécifique.

On pourra se référer plus précisément aux documents de l'art antérieur cités précédemment pour la réaction d'oxydéshydrogénation des acides carboxyliques saturés, pour mettre en oeuvre la méthode la plus appropriée pour préparer les catalyseurs à base de phosphate de fer dopés utilisables pour le procédé de l'invention.

Il est aussi possible de préparer les catalyseurs pour le procédé selon l'invention par variation à partir des méthodes générales décrites dans les documents suivants :
- JM Millet, JC Védrine et G. Hecquet, dans « New Developments in Selective Oxidation », (1990), page 833, Elsevier Science Publishers, G. Centi and F Trifiro Eds.
- Ai et al. J. Mol. Catal. 89 (1984) 371-381
- JM Millet, Catalysis Review 40 :1, page 1-38
- AM Beale et al., J. Mat. Chem. 12 (2002) 3064-3072

Le procédé selon l'invention peut être mis en oeuvre en phase gaz ou en phase liquide, de préférence en phase gaz. Lorsque la réaction de déshydratation est réalisée en phase gaz, différentes technologies de procédé peuvent être utilisées, à savoir procédé en lit fixe, procédé en lit fluidisé ou procédé en lit fluidisé circulant. On peut utiliser aussi des réacteurs de type échangeurs à plaques, comme ceux décrits par exemple dans les documents EP 995491 ou EP 1147807.

La déshydratation du glycérol peut aussi être mise en oeuvre en phase liquide dans un réacteur classique pour réaction en phase liquide, mais aussi dans un réacteur de type distillation catalytique. Etant donné la différence importante entre les points d'ébullition du glycérol (280°C) et de l'acroléine (53°C), on peut aussi envisager un procédé en phase liquide à une température relativement basse qui permet une distillation en continu de l'acroléine produite. La réaction est déplacée en permanence limitant ainsi les réactions consécutives sur l'acroléine dans un réacteur continu à déplacement d'équilibre.

Les conditions expérimentales de la réaction en phase gaz sont de préférence une température comprise entre 180°C et 500°C, de préférence entre 250 à 400°C et une pression comprise entre 1 et 5 bars. En phase liquide, la réaction est mise en oeuvre de préférence à une température comprise entre 150°C et 350°C et une pression pouvant aller de 3 à 70 bars.

Dans le procédé de l'invention, on utilise généralement une solution aqueuse de glycérol de concentration allant de 20 % à 99 % en poids dans le réacteur, de préférence entre 30 % et 80 %.

La solution de glycérol peut être utilisée sous forme liquide ou sous forme gazeuse, de préférence sous forme phase gaz.

Nous allons décrire maintenant un mode de réalisation préféré de l'invention. Le procédé de préparation d'acroléine à partir de glycérol consiste à envoyer un mélange contenant au moins du glycérol, de l'eau, de l'oxygène ou un gaz contenant de l'oxygène, et le cas échéant un gaz inerte et/ou des gaz de recyclage, en phase gaz, sur un lit d'un système catalytique tel que défini précédemment, maintenu à une température de réaction comprise entre 180 et 500°C.

La charge envoyée dans le réacteur peut être préalablement chauffée à une température de préchauffage de l'ordre d'environ 180°C à 350 °C.

On opère à une pression de l'ordre de la pression atmosphérique et, plus précisément de préférence, à une pression légèrement supérieure.

La quantité d'oxygène est choisie de façon à être en dehors du domaine d'inflammabilité en tout point de l'installation. Le rapport molaire entre l'oxygène moléculaire et le glycérol est généralement de l'ordre de 0,1 à 1,5, de préférence de 0,5 à 1,0.

Un autre paramètre réside dans la concentration du glycérol dans la charge. Exprimée en moles pour cent, la concentration du glycérol varie largement de 0,1 à 20. Comme il est courant dans des réactions de ce type, le rendement du produit désiré est une fonction inverse de la concentration. Du point de vue de l'obtention d'un débit raisonnable combiné avec un rendement acceptable, la concentration du glycérol dans la charge est d'environ 3 à 16 moles %. La concentration est contrôlée par la quantité d'eau et du gaz inerte présente dans le courant d'alimentation. Le diluant gazeux préféré est l'azote bien que d'autres gaz tels que le dioxyde de carbone, hélium, argon, etc., soient également appropriés. Bien entendu, si la concentration désirée du glycérol le permet, l'air représente un oxydant dilué convenable.

La durée de contact exprimée en secondes est le rapport entre le volume du lit de catalyseur et le volume des réactifs gazeux envoyés par seconde. Les conditions moyennes de température et de pression existant dans un lit peuvent varier selon la nature du catalyseur, la nature du lit catalytique et la dimension du catalyseur. En général, la durée de contact est de 0,1 à 20 secondes et de préférence de 0,3 à 15 secondes.

Les catalyseurs utilisés dans le procédé de la présente invention permettent d'atteindre des rendements élevés en acroléine avec des taux de conversion extrêmement élevés pouvant atteindre dans certains cas jusqu'à 100% du glycérol. Ces résultats sont dus au fait que ces catalyseurs ont l'avantage de promouvoir un procédé de déshydratation à déroulement régulier et aisément contrôlable quant aux températures réactionnelles et aux durées de contact. Les réactifs peuvent être introduits sur le catalyseur, que ceux-ci soit déjà totalement ou seulement partiellement pré-mélangés, ou peuvent être introduits individuellement.

L'alimentation en différents réactifs, appliquée à un réacteur à lit fixe ou à lit fluidisé peut se faire de façon individuelle ou déjà sous la forme de pré-mélanges. Il est possible également d'introduire une partie de l'air ou éventuellement la totalité du glycérol ou seulement une partie de ce glycérol à la base du réacteur et d'alimenter successivement les parties restantes du réactif en un ou plusieurs points intermédiaires du lit catalytique. Lorsque la réaction est effectuée selon les techniques de lits catalytiques fixes, de tels lits peuvent être obtenus selon les méthodes connues par disposition du catalyseur dans les tubes d'un réacteur à faisceau et par élimination de leur chaleur réactionnelle à l'aide de fluides convenables circulant à l'extérieur des tubes, ces fluides pouvant par exemple et le plus généralement consister en des mélanges de sels fondus. Il est également possible d'opérer dans un réacteur en plusieurs étapes réactionnelles adiabatiques séparées par des zones de refroidissement du mélange réactionnel.

Selon un mode de réalisation particulier de l'invention, il est possible de disposer en amont du système catalytique à base de phosphate de fer dopé un premier lit de catalyseur actif, ou un premier réacteur permettant d'effectuer la réaction de déshydratation du glycérol en acroléine. Le mélange gazeux réactionnel est adressé ainsi à un premier catalyseur au contact duquel la réaction de déshydratation du glycérol s'effectue au moins partiellement en conduisant généralement à des composés secondaires tels que le propanaldéhyde. Le mélange réactionnel ainsi obtenu est ensuite en contact avec le système catalytique sur lequel la réaction de déshydratation du glycérol non réagi peut se poursuivre en même temps que la transformation du propanadéhyde en acroléine. Le premier lit catalytique peut opérer à une température plus faible que le second lit catalytique, optimisant ainsi le bilan énergétique du procédé. L'acroléine obtenu selon ce mode de réalisation contient une quantité minimisée en propanaldéhyde, ce qui élargit son champ d'application. Cette configuration de réacteurs est possible selon différentes technologies, par exemple en lit fixe adiabatique, mais aussi en lit fixe multitubulaire, ou encore par exemple en lit fluidisé compartimenté. Elle est aussi possible dans le cas où le premier réacteur fonctionne en phase liquide, et le second contenant le catalyseur à base de phosphate de fer fonctionne en phase gaz.

Sur une longue durée d'utilisation, le système catalytique peut tendre à être moins efficace tant en ce qui concerne le taux de conversion que la sélectivité. Le catalyseur sera soumis alors à une étape de régénération, par exemple selon la méthode décrite dans le document FR 2 498 476 qui consiste à soumettre le catalyseur à base de phosphate de fer à une atmosphère fortement oxydante à environ 350°C pendant au moins une durée de deux heures, puis à le soumettre à une atmosphère réductrice à environ la même température.

D'autres méthodes de régénération peuvent être utilisées, notamment celle décrite dans le document EP 263 005 consistant à régénérer le catalyseur par addition d'un composé phosphoré, ou celle décrite dans le document US 3,716,545 consistant à ajouter du phosphore durant le procédé de façon à augmenter ou maintenir l'activité du catalyseur.

On ne sortirait pas du cadre de la présente invention si le procédé est mis en oeuvre en présence d'un gaz contenant du propylène, comme décrit dans la demande WO 07/090990.

L'invention porte aussi sur un procédé de préparation d'acide acrylique à partir de glycérol comprenant une première étape de préparation d'acroléine selon le procédé décrit précédemment, et une étape d'oxydation de l'acroléine en acide acrylique.

L'invention porte aussi sur un procédé de préparation d'acide acrylique à partir de glycérol comprenant une première étape de préparation d'acroléine selon le procédé décrit précédemment et une seconde étape d'oxydation de l'acroléine en acide acrylique, dans lequel on met en oeuvre une étape intermédiaire de condensation partielle de l'eau et des sous-produits lourds issus de la première étape, telle qu'elle est décrite dans la demande WO 08/087315.

L'invention porte aussi sur un procédé de préparation d'acrylonitrile à partir de glycérol comprenant une première étape de préparation d'acroléine selon le procédé décrit précédemment, et une étape d'ammoxydation de l'acroléine en acrylonitrile.

Les systèmes catalytiques utilisables dans le procédé de la présente invention peuvent également être mis en oeuvre pour favoriser une réaction d'oxydéshydratation du glycérol en acide acrylique, l'homme du métier déterminera dans ce cas les conditions opératoires à utiliser.

Les exemples suivants illustrent la présente invention sans toutefois en limiter la portée.

### EXEMPLES

Dans les exemples qui suivent, on entend par sélectivité en acroléine (en %) le rapport nombre de moles d'acroléine formées / nombre de moles de glycérol ayant réagi * 100, le rendement en acroléine (en %) est le rapport nombre de moles d'acroléine formées / nombre de moles de glycérol introduites * 100, les gaz réactionnels étant analysés par chromatographie en phase gazeuse.

### Exemple 1 (comparatif)

En utilisant le catalyseur Fe₁P₂ préparé selon l'exemple 1 du document FR 2 245 604 sous la forme d'un lit fixe dans un réacteur alimenté par un mélange gazeux constitué de glycérol, d'air, vapeur d'eau, azote, dans les proportions molaires 1/3,13/15/55 à une température de 320°C, et une durée de contact de 2 s, on peut obtenir une sélectivité en acroléine de 65 %.

### Exemple 2

En utilisant le catalyseur préparé selon l'exemple 8 du document FR 2 245 604, répondant à la formule empirique Fe₁P_{1,84}Cs_{0,66}, sous la forme d'un lit fixe dans un réacteur alimenté par un mélange gazeux constitué de glycérol, d'air, vapeur d'eau, azote, dans les proportions molaires 1/3,1/25/55 à une température de 380°C, et une durée de contact de 0,5 s , on peut obtenir une sélectivité en acroléine de 75 %.

### Exemple 3

En utilisant le catalyseur préparé selon l'exemple 15 du document FR 2 245 604, répondant à la formule empirique Fe₁P_{1,4}Sr_{0,25}, sous la forme d'un lit fixe dans un réacteur alimenté par un mélange gazeux constitué de glycérol, d'air, vapeur d'eau, azote, dans les proportions molaires 1/3,6/25/55 à une température de 380°C, et une durée de contact de 1 s, on peut obtenir une sélectivité en acroléine de 70 %.

### Exemple 4

Un réacteur tubulaire contenant un lit fixe de catalyseur répondant à la formule Fe_{1,0}Cs_{0,1}P_{1,26}Oₓ/SiO₂ à 320 °C est alimenté à un débit d'alimentation de 8,5 ml/h de glycérol, 30,0 ml/h, d'eau et 120,0 ml/min d'air. On peut obtenir dans ces conditions un taux de conversion de 95 % en glycérol et un rendement de 76% en acroléine.

### Exemple 5

On utilise un catalyseur préparé selon l'exemple 16 du document FR 2 245 604, répondant à la formule empirique Fe₁P_{1,84}K_{0,66}. La réaction de déshydratation est effectuée dans un réacteur contenant ce catalyseur sous la forme d'un lit fluidisé et alimenté par un mélange gazeux constitué de glycérol, d'air et de vapeur d'eau dans les proportions molaires 1/2,5/44 à une température de 330°C, et une durée de contact de 1,3 s. Par analyse chromatographique en phase gazeuse des gaz réactionnels, on obtient un taux de conversion du glycérol de 87% et une sélectivité en acroléine de 71%.

### Exemple 6

Un catalyseur répondant à la formule empirique Fe₁P_{1,44}Ag_{0,18}Oₓ est préparé de la façon suivante : On dissout 103,2 g de nitrate de fer nona-hydraté avec 7,5 g de nitrate d'argent dans 200 ml d'eau distillée. On ajoute à cette solution 35,8 g d'acide phosphorique concentré avec 30 cm³ de gel de silice contenant 20% de SiO₂. On agite la solution à 85°C jusqu'à ce que la majeure partie de l'eau soit évaporée. On sèche la pâte obtenue à 120°C jusqu'à ce que l'eau apparaisse complètement évaporée, puis on poursuit le chauffage à 150°C pendant une nuit. Le produit sec est ensuite calciné à 450°C pendant 16h, puis à 520°C pendant 2h.

On introduit 15 cm³ de ce catalyseur dans un réacteur pour former un lit fixe maintenu à une température de 385°C. Le réacteur est alimenté avec un débit de 41 l/h d'un mélange gazeux constitué de glycérol, d'oxygène, de vapeur d'eau et d'azote, dans les proportions molaires 1/1/25/20. On obtient un taux de conversion du glycérol de 86% et une sélectivité en acroléine de 68%.

### Exemple 7

On dissout 404,4 g de nitrate de fer nona-hydraté avec 127,2 g d'acide phosphorique à 85% (poids de H₃PO₄) dans 400 ml d'eau distillée. On ajoute à la solution obtenue 100 ml de sol de silice contenant 40% de dioxyde de silicium (Ludox AS40). On agite ensuite la solution obtenue à 85 °C jusqu'à ce que la majeure partie de l'eau soit évaporée. On sèche encore la pâte obtenue à 120 °C jusqu'à ce qu'on puisse la fragmenter puis on poursuit le séchage pendant 12 h. On calcine le mélange séché à 460°C pendant 6 h.

Le solide obtenu est alors imprégné avec une solution d'acide chloroplatinique. On effectue une imprégnation à humidité naissante.

La formule empirique de la composition calcinée est la suivante: FeP_{1,3}Si_{0,67}Pt_{0,02}Oₓ.

Le catalyseur sous forme de grains de 65 à 150 microns dans un lit fixe est alimenté par un mélange gazeux de glycérol, d'air, de vapeur d'eau et d'azote dans les proportions molaires 3/20/60/15 à une température de 300 °C et un temps de contact de 2 secondes. On obtient un rendement en acroléine de 53 %.

## Revendications

1. Procédé de fabrication d'acroléine à partir de glycérol **caractérisé en ce que** l'on effectue la réaction de déshydratation du glycérol en présence d'un système catalytique comprenant un catalyseur répondant à la formule générale FePₓM'_{y}M"_{w}O_{z} dans laquelle :
- M' représente au moins l'un des éléments suivants: métal alcalin, métal alcalino-terreux, Al, B, Co, Ni, Sn, Sb, Mn, Ag, seuls ou en mélange, M" représente au moins l'un des éléments suivants: Pt, Rh, seuls ou en mélange,
- x allant de 0,2 à 3,0, bornes comprises, de préférence allant de 0,9 à 2,0
- y allant de 0,01 à 2,0, bornes comprises
- w allant de 0,0 à 0,1, bornes comprises
- z est la quantité d'oxygène liée aux autres éléments et correspondant à leur état d'oxydation.

2. Procédé selon la revendication 1 **caractérisé en ce que** dans la formule FePₓM'_{y}M"_{w}O_{z}, M'représente Cs, Al, B, Ag.

3. Procédé selon la revendication 1 ou 2 **caractérisé en ce que** le système catalytique comprend en outre un support tel que silice, alumine, oxyde de titane, carbure de silicium, mélange silice-alumine, silicates, borates, carbonate.

4. Procédé selon l'une des revendications précédentes **caractérisé en ce qu'**un premier lit de catalyseur actif ou un premier réacteur permettant d'effectuer la réaction de déshydratation du glycérol en acroléine est placé en amont du système catalytique comprenant le catalyseur de formule FePₓM'_{y}M"_{w}O_{z} précitée.

5. Procédé selon l'une quelconque des revendications précédentes **caractérisé en ce que** l'on fait passer un mélange contenant au moins du glycérol, de l'eau, de l'oxygène ou un gaz contenant de l'oxygène, et le cas échéant un gaz inerte et/ou des gaz de recyclage, en phase gaz, sur le système catalytique maintenu à une température de réaction comprise entre 180 et 500°C.

6. Procédé de préparation d'acide acrylique à partir de glycérol comprenant une première étape de préparation d'acroléine selon le procédé de l'une des revendications 1 à 5 et une étape d'oxydation de l'acroléine en acide acrylique.

7. Procédé selon la revendication 6 **caractérisé en ce que** l'on met en oeuvre une étape intermédiaire de condensation partielle de l'eau et des sous-produits lourds issus de la première étape.

8. Procédé de préparation d'acrylonitrile à partir de glycérol comprenant une première étape de préparation d'acroléine selon le procédé de l'une des revendications 1 à 5 et une étape d'ammoxydation de l'acroléine en acrylonitrile.

## Patentansprüche

1. Verfahren zur Herstellung von Acrolein aus Glycerol, **dadurch gekennzeichnet, dass** die Dehydratisierungsreaktion von Glycerol in Gegenwart eines katalytischen Systems durchgeführt wird, das einen Katalysator umfasst, der der allgemeinen Formel FePₓM'_{y}M"_{w}O_{z} entspricht, worin:
- M' für mindestens eines der folgenden Elemente steht: Alkalimetall, Erdalkalimetall, Al, B, Co, Ni, Sn, Sb, Mn, Ag, allein oder als Gemisch, M" für mindestens eines der folgenden Elemente steht: Pt, Rh, allein oder als Gemisch,
- x zwischen 0,2 und 3,0, einschließlich der Grenzwerte, bevorzugt zwischen 0,9 und 2,0 liegt,
- y zwischen 0,01 und 2,0, einschließlich der Grenzwerte, liegt,
- y zwischen 0,0 und 0,1, einschließlich der Grenzwerte, liegt,
- z die Menge an Sauerstoff ist, die an andere Elemente gebunden ist und deren Oxidationszahl entspricht.

2. Verfahren nach Anspruch 1, **dadurch gekennzeichnet, dass** in der Formel FePₓM'_{y}M"_{w}O_{z} M' für Cs, Al, B, Ag steht.

3. Verfahren nach Anspruch 1 oder 2, **dadurch gekennzeichnet, dass** das katalytische System ferner einen Träger umfasst, wie beispielsweise Siliziumdioxid, Aluminiumoxid, Titanoxid, Siliciumcarbid, ein Gemisch aus Siliciumdioxid-Aluminiumoxid, Silicate, Borate, Carbonat.

4. Verfahren nach einem der vorergehenden Ansprüche, **dadurch gekennzeichnet, dass** eine erste aktive Katalysatorschicht oder ein erster Reaktor, der es ermöglicht, die Dehydratisierungsreaktion von Glycerol zu Acrolein auszuführen, stromaufwärts des katalytischen Systems, das den Katalysator der vorgenannten Formel FePₓM'_{y}M"_{w}O_{z} umfasst, angeordnet ist.

5. Verfahren nach einem der vorhergehenden Ansprüche, **dadurch gekennzeichnet, dass** ein Gemisch, das mindestens Glycerol, Wasser, Sauerstoff oder ein Gas, das Sauerstoff enthält, und gegebenenfalls ein Inertgas und/oder Recyclinggase in Gasphase enthält, durch das katalytische System hindurchgeleitet wird, das auf einer Reaktionstemperatur im Bereich zwischen 180 und 500 °C gehalten wird.

6. Verfahren zur Herstellung von Acrylsäure aus Glycerol, umfassend einen ersten Schritt zur Herstellung von Acrolein nach dem Verfahren nach einem der Ansprüche 1 bis 5 und einen Schritt zur Oxidation des Acroleins zu Acrylsäure.

7. Verfahren nach Anspruch 6, **dadurch gekennzeichnet, dass** ein Zwischenschritt zur teilweisen Kondensation des Wassers und der schweren Nebenprodukte, die aus dem ersten Schritt stammen, durchgeführt wird.

8. Verfahren zur Herstellung von Acrylnitril aus Glycerol, umfassend einen ersten Schritt zur Herstellung von Acrolein nach dem Verfahren nach einem der Ansprüche 1 bis 5 und einen Schritt zur Ammoxidation des Acroleins zu Acrylnitril.

## Claims

1. Process for manufacturing acrolein from glycerol, **characterized in that** the reaction for dehydration of glycerol is carried out in the presence of a catalyst system comprising a catalyst corresponding to the general formula FePₓM'_{y}M"_{w}O_{z} in which:
- M' represents at least one of the following elements: alkali metal, alkaline-earth metal, Al, B, Co, Ni, Sn, Sb, Mn, Ag, alone or as a mixture, M" represents at least one of the following elements: Pt or Rh, alone or as a mixture;
- x ranging from 0.2 to 3.0, limits included, preferably ranging from 0.9 to 2.0 ;
- y ranging from 0.01 to 2.0, limits included;
- w ranging from 0.0 to 0.1, limits included; and
- z is the amount of oxygen bound to the other elements and corresponding to their oxidation state.

2. Process according to Claim 1, **characterized in that** in the formula FePₓM'_{y}M"_{w}O_{z}, M' represents Cs, Al, B or Ag.

3. Process according to Claim 1 or 2, **characterized in that** the catalyst system additionally comprises a support such as silica, alumina, titanium oxide, silicon carbide, silica/alumina mixture, silicates, borates or carbonate.

4. Process according to one of the preceding claims, **characterized in that** a first active catalyst bed or a first reactor enabling the dehydration reaction of glycerol to acrolein to be carried out is placed upstream of the catalyst system comprising the aforementioned catalyst of formula FePₓM'_{y}M"_{w}O_{z}.

5. Process according to one of the preceding claims, **characterized in that** a mixture containing at least glycerol, water, oxygen or an oxygen-containing gas, and where appropriate an inert gas and/or recycle gases, in the gas phase, is passed over the catalyst system kept at a reaction temperature between 180 and 500°C.

6. Process for preparing acrylic acid from glycerol comprising a first step of preparing acrolein according to the process of one of Claims 1 to 5 and a step of oxidizing acrolein to acrylic acid.

7. Process according to Claim 6, **characterized in that** an intermediate step of partial condensation of the water and of the heavy by-products from the first step is carried out.

8. Process for preparing acrylonitrile from glycerol comprising a first step of preparing acrolein according to the process of one of Claims 1 to 5 and a step of ammoxidation of the acrolein to acrylonitrile.
